(19) Europäisches Patentamt / European Patent Office / Office européen des brevets

(11) **EP 3 924 022 B1**

(12) **EUROPÄISCHE PATENTSCHRIFT**

(45) Veröffentlichungstag und Bekanntmachung des Hinweises auf die Patenterteilung:
**03.04.2024   Patentblatt 2024/14**

(21) Anmeldenummer: **20708038.3**

(22) Anmeldetag: **13.02.2020**

(51) Internationale Patentklassifikation (IPC):
**A61M 11/00** (2006.01)    **A61M 15/06** (2006.01)
**A24F 42/20** (2020.01)    **B05B 1/26** (2006.01)
**B05B 1/02** (2006.01)    **A61M 16/08** (2006.01)
**A61F 9/00** (2006.01)

(52) Gemeinsame Patentklassifikation (CPC):
**B65D 83/205; A24F 42/20; A61M 11/001;
A61M 11/007; A61M 11/008; A61M 11/02;
A61M 15/009; A61M 15/06; B05B 1/14;
B05B 1/262; B05B 7/0012; B65D 83/753;
B65D 83/754;** A61F 9/0008; A61M 11/003;   (Forts.)

(86) Internationale Anmeldenummer:
**PCT/EP2020/053782**

(87) Internationale Veröffentlichungsnummer:
**WO 2020/165356 (20.08.2020 Gazette 2020/34)**

(54) **TRAGBARE VORRICHTUNG ZUM VERABREICHEN EINER PHYSIOLOGISCH WIRKSAMEN FLÜSSIGKEIT**

PORTABLE DEVICE FOR ADMINISTERING A PHYSIOLOGICALLY ACTIVE LIQUID

DISPOSITIF PORTABLE POUR L'ADMINISTRATION D'UN LIQUIDE PHYSIOLOGIQUEMENT ACTIF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.02.2019   DE 202019000718 U
02.10.2019   DE 202019004067 U**

(43) Veröffentlichungstag der Anmeldung:
**22.12.2021   Patentblatt 2021/51**

(73) Patentinhaber: **Werrta GmbH Düsen- und Zerstäubungstechnik
14089 Berlin (DE)**

(72) Erfinder:
• **RENTSCH, Rüdiger
14089 Berlin (DE)**
• **ETZOLD, Mathias
13581 Berlin (DE)**

(74) Vertreter: **Kehl, Ascherl, Liebhoff & Ettmayr Patentanwälte Partnerschaft mbB
Emil-Riedel-Straße 18
80538 München (DE)**

(56) Entgegenhaltungen:
WO-A1-2017/167610    DE-A1-102005 030 803
US-A- 3 838 686    US-A- 4 368 850
US-A1- 2015 174 595    US-A1- 2018 214 894

• S. P. Lin ET AL: "DROP AND SPRAY FORMATION FROM A LIQUID JET", Annual Review of Fluid Mechanics, vol. 30, no. 1, 1 January 1998 (1998-01-01), pages 85-105, XP055009750, ISSN: 0066-4189, DOI: 10.1146/annurev.fluid.30.1.85

(52) Gemeinsame Patentklassifikation (CPC): (Forts.)
A61M 15/0015; A61M 16/0808; A61M 2205/3334;
A61M 2205/8225; A61M 2205/8281; A61M 2207/00;
B05B 11/1052; B65D 83/62; B65D 83/64

**Beschreibung**

Die vorliegende Erfindung betrifft eine tragbare Vorrichtung zum Verabreichen einer physiologisch wirksamen Flüssigkeit, insbesondere als Aerosol.

Aus dem Stand der Technik sind Inhalationsvorrichtungen zum Verabreichen von Aerosolen bekannt, beispielsweise medizinische Inhalatoren oder auch Vorrichtungen zur Raucherentwöhnung oder als Rauchwarenersatz, wie E-Zigaretten.

Inhalatoren sind hinlänglich bekannt als Vorrichtungen, welche eine Flüssigkeit zerstäuben und dem Anwender die Inhalation des durch die Zerstäubung entstehenden Aerosols ermöglichen. Die hierbei verwendeten Inhalationsflüssigkeiten bestehen zumeist zu einem überwiegenden Anteil aus Wasser, das mit einem oder mehreren medizinischen Wirkstoffen versetzt sein kann.

Übliche Inhalatoren weisen einen Speicher für die Inhalationsflüssigkeit, einen Zerstäuber, durch welchen die Inhalationsflüssigkeit zerstäubt wird, sowie einen Applikator auf, welcher eine mehr oder minder zielgerichtete Zuführung des durch die Zerstäubung entstehenden Aerosols zu den Luftwegen des Anwenders ermöglicht. Beispielsweise kann es sich beim Applikator um eine über Nase und/oder Mund zu legende Maske oder eine an einem Ende vom Mund umschließbare Röhre, meist mit rundem oder ovalen Querschnitt, handeln.

Wesentlicher Bestandteil des Zerstäubers ist in der Regel eine Düsenanordnung mit einer oder mehreren Düsenöffhungen, aus welcher bzw. welchen die Inhalationsflüssigkeit austritt, hierbei Tröpfchen und zusammen mit dem umgebenden Gas, in der Regel Luft, ein Aerosol bildet. Neben der Energie, die benötigt wird, die Inhaltionsflüssigkeit durch die Düsenanordnung zu bewegen, muss auch noch die Oberflächenergie für die Oberflächen der sich bildenden Tröpfchen aufgewendet werden. Je kleiner die entstehenden Tröpfchen, desto größer ist die Gesamtoberfläche des entstehenden Aerosols und entsprechend der Energieaufwand.

Inhalatoren können mit einem komprimierten Treibmittel, z.B. Druckluft, betrieben werden. Technisch aufwendigere Inhalatoren arbeiten häufig mit anderen oder zusätzlichen Energiequellen als komprimiertem Treibmittel, beispielsweise elektromechanisch oder mittels Ultraschallzerstäubung. Aufgrund des technischen Aufwands und der damit verbundenen Kosten ist das Anwendungsspektrum derartiger Inhalatoren begrenzt. Insbesondere lassen sie sich nicht einfach einstecken um bei Bedarf, beispielsweise während sportlicher Betätigung, auf Reisen, oder im normalen Alltagsgeschehen, sofort zur Verfügung zu stehen.

Nach dem Venturiprinzip arbeitende Inhalatoren verwenden einen Gasstrom, der Flüssigkeit in einer Zweistromdüse mit sich reißt. Durch Auftreffen des Zweistoffstrohms auf eine Prallplatte wird die Flüssigkeit weiter vernebelt. Der Betrieb derartiger Inhalatoren erfordert hohe Gasurchsätze und eignet sich daher nicht für portable Anwendung mit langer Inhalationsdauer.

Aus WO 2016/184761 A1 ist ein transportabler Inhalator bekannt, der mit einem Flüssigkeitsspeicher für Inhalationsflüssigkeit ausgestattet ist, der durch Beaufschlagung mit Treibgas, Druckluft oder durch eine vorgespannte Federeinrichtung unter Druck steht. Dabei kann der Flüssigkeitsspeicher ein definiertes Volumen aufweisen, so dass entsprechend dem Flüssigkeitsaustrag Luft nachströmt, oder aber unbelüftet sein und ein über einen Beutel oder einen Schleppkolben variables Volumen aufweisen. Die Lagerung von Treibgas im Flüssigkeitsspeicher wird in WO 2016/184761 A1 als mögliche Variante ebenso beschrieben wie eine manuelle Pumpeinrichtung. Dabei werden keine Angaben zur Höhe der eingesetzten Drücke und zur Bemessung der Volumina von Flüssiggkeitsspeicher und Überdruck-Luftspeicher gemacht. Die Flüssigkeit tritt durch eine Düsenplatte aus, die eine Vielzahl paralleler Düsenöffnungen aufweist. Hierdurch soll eine möglichst monodisperse Tröpfchengrößenverteilung erreicht werden.

Die Patentschrift DE 10 2014 207 657 B3 offenbart ein Verfahren zur Erzeugung eines Flüssigkeitssprays, bei welchem ein Prallelement mit einer Erhebung vorgesehen ist, auf welches wechselweise ein kontinuierlicher Flüssigkeitsstrahl und ein Tröpfchenstrahl auftreffen. Der Tröpfchenstrahl wird durch Einbringen von Vibrationen mittels eines Piezo-Elements erzeugt, werden keine Vibrationen eingebracht, ergibt sich der kontinuierliche Strahl.

Aus US 4,368,850 ist ein Aerosolgenerator bekannt, bei dem Flüssigkeit eine Düse gedrückt und rechtwinklig zur Düsenachse Luft zugeführt wird. Dabei tritt direkt aus der Düse ein Spray mit überwiegend groben Partikeln im Kern und überwiegend feinen Partikeln im Außenbereich aus. Grobe Partikeln werden abgeschieden durch Umlenkung sowie durch Auftreffen des Sprays auf innenliegende Wände und ein der Düse gegenüberliegendes eiförmiges Hindernis. US 3,838,686 offenbart ein gegenüber einer Düse angeordnetes konisches Hindernis. Auch hier erzeugt die Düse einen Spraykegel mit überwiegend groben Tröpfchen im Zentrum und überwiegend feinen Tröpfchen im Außenbereich. Die groben Tröpfchen im Inneren, die auf das Hindernis treffen, unterliegen größtenteils Koaleszenz, die feinen Tröpfchen in Außenbereich passieren das Hindernis.

Wesentliche Bedeutung kommt der Größe bzw. Größenverteilung der Tröpfchen zu, die bei der Zerstäubung im Inhalator entstehen. Denn abhängig von ihrer Größe gelangen Tröpfchen eines inhalierten Aerosols gegebenenfalls lediglich in die oberen Luftwege bzw. die Bronchien, erst unterhalb eines Tröpfchenurchmessers von etwa 10 $\mu$m sind Aerosoltröpfchen lungengängig, je nach Zieldisposition kann eine Tröpfchengröße zwischen 2 und 5 $\mu$m als ideal gelten.

Es hat sich erwiesen, dass herkömmliche Inhalatoren nur dann hinreichend kleine Tröpfchen über einen ausreichend langen Zeitraum erzeugen können, wenn die Inhalatoren entweder mit einer stationär Druckluftquelle betrieben werden oder als elektrisch betriebene Einheiten ausgeführt sind. Insbesondere übliche Sprühdosen, welche standardmäßig mit einem Fülldruck von 13,2 bar (1,32 MPa) beaufschlagt werden, eignen sich bislang kaum für den Einsatz als Inhalator, insbesondere wenn die Wirkstoffe in der Inhalationsflüssigkeit direkt in die Lunge gelangen sollen.

Bei Inhalationsvorrichtungen zur Gabe kleiner Nikotindosen, landläufig als E-Zigaretten bekannt, erfolgt üblicherweise kein rein mechanischer Austrag der nikotinhaltigen Flüssigkeit, sondern die Flüssigkeit wird erhitzt, so dass kondensierte Dampftröpchen inhaliert werden. Beim Erhitzen werden jedoch auch unerwünschte chemische Prozesse in Gang gesetzt, wodurch unerwünschte, auch toxische, Verbindungen im Tröpchenkondensat enthalten sein können.

Aufgabe der vorliegenden Erfindung ist es vor diesem Hintergrund, eine tragbare Vorrichtung zum Verabreichen einer physiologisch wirksamen Flüssigkeit zu schaffen, die mobil verwendet werden kann und dabei während ihrer gesamten Nutzungsdauer hinreichend kleine Aerosoltröpchen liefert.

Gemäß einem Aspekt der vorliegenden Erfindung wird eine tragbare Vorrichtung zum Verabreichen einer physiologisch wirksamen Flüssigkeit geschaffen, welche einen Behälter zum Aufnehmen der Flüssigkeit, Druckbeaufschlagungsmittel zum Beaufschlagen der Flüssigkeit mit Druck, einen Zerstäuber zum Zerstäuben der Flüssigkeit und einen Applikator zum Verabreichen zerstäubter Flüssigkeit aufweist. Der Zerstäuber weist mindestens eine Düse, durch welche Flüssigkeit aus dem Behälter ausstoßbar ist, und ein funktional mit der Düse kombiniertes düsenaustrittsseitiges Prallelement (beispielsweise eine Prallplatte) zur Prallzerstäubung auf und ist dabei so ausgelegt, dass in einem mit dem Druckbeaufschlagungsmittel erzeugbaren Druckbereich aus der Düse austretender Flüssigkeitsstrahl vor dem Auftreffen auf das Prallelement in freiem Zerfall in Tröpchen zerfällt.

Eine gezielte vibratorische Anregung des Strahls, eine Strahlunterbrechung oder Modulierung des Flüssigkeitsausstoßes durch die Düse ist also erfindungsgemäß nicht vonnöten.

Eine solche Auslegung kann empirisch durch einfache Auslegungsversuche erfolgen. Orientieren kann sich der Fachmann dabei an der folgenden Beziehung

$$Z = D \ln \frac{D}{2C} \sqrt{We}[1 + 3Oh]$$

für die Strahlaufbruchlänge Z, worin $We = \frac{\rho U^2 D}{\sigma}$ die Weberzahl und $Oh = \frac{\eta}{\sqrt{D\sigma\rho}}$ die Ohnesorgezahl bezeichnet mit

Z    Strahlaufbruchlänge in m
D    engster Düsendurchmesser in m
C    Anfangsstörung des Strahlzerfalls in m
$\rho$    Dichte der physiologisch wirksamen Flüssigkeit in kg/m$^3$
$\sigma$    Oberflächenspannung der physiologisch wirksamen Flüssigkeit in N/m
$\eta$    Viskosität der physiologisch wirksamen Flüssigkeit in Pa s
U    die Austrittsgeschwindigkeit des Flüssigkeitsstrahls aus der Düse

Die Anfangsstörung des Strahlzerfalls C ist in der Regel eine Unbekannte, für die vorliegende Erfindung hat sich jedoch erwiesen, dass für den dimensionslose Faktor

$$\ln \frac{D}{2C}$$

üblicherweise ein Wert wischen 10 und 15, zumeist von 12 bis 13 angenommen werden kann.

Bei der Prallzerstäubung von durch freien Strahlenzerfall entstehende Tröpchen in einer erfindungsgemäßen Vorrichtung sind gemäß Versuchswerten beispielsweise

Bei einem Düsendurchmesser von D = 15 $\mu$m und Drücken von 15 bis 25 bar:

$D_{v90} \approx 10 \ \mu$m
$D_{v50} \approx 5$ bis 7 $\mu$m
$D_{v10} \approx 3 \ \mu$m

Bei einem Düsendurchmesser von D = 10 $\mu$m und Drücken von ca. 25 bar:

$D_{v90} \approx 4\ \mu m$
$D_{v50} \approx 1\ bis\ 2\ \mu m$
$D_{v10} \approx 1\ \mu m$

Die Durchmesserangaben in den obigen Beispielen sind dabei wie folgt zu verstehen:

$D_{v10}$ 10% des Flüssigkeitsvolumens des Aerosols besteht aus Tröpfchen kleiner als $D_{v10}$
$D_{v50}$ 50% des Flüssigkeitsvolumens des Aerosols besteht aus Tröpfchen kleiner als $D_{v50}$
$D_{v90}$ 90% des Flüssigkeitsvolumens des Aerosols besteht aus Tröpfchen kleiner als $D_{v90}$

Gemäß einer besonders vorteilhaften Weiterbildung weist die Vorrichtung eine Auffangvorrichtung zum Auffangen eines vom Prallelement abtropfenden oder abfließenden Flüssigkeitsüberschusses auf. Der erfindungsgemäße Einsatz eines Prallelements führt stets zu einem gewissen Anteil ungenutzt vom Prallelement abtropfender Flüssigkeit. Für die Handhabung eines tragbaren Inhalators oder eine andere tragbare Vorrichtung zum Applizieren eines Aerosols ist es von großem Vorteil, wenn der Nutzer nicht durch unkontrolliert aus der Vorrichtung tropfender Flüssigkeit beeinträchtigt sondern diese kontrolliert zurückgehalten wird.

Gemäß einer vorteilhaften Ausführungsform können die Auffangvorrichtung und der Applikator in ein gemeinsames Bauteil integriert sein.

Gemäß einer weiteren vorteilhaften Ausführungsform können die Auffangvorrichtung und das Prallelement in ein gemeinsames Bauteil integriert sein.

Gemäß einer weiteren vorteilhaften Ausführungsform kann die Auffangvorrichtung ein Reservoir aufweisen. Vorteilhafterweise kann das Reservoir ein, auswechselbares oder nicht wechselbar integriertes, saugfähiges Material, beispielsweise ein Vlies, Schwämmchen, Zeolith oder dgl. aufweisen.

Gemäß einer weiteren vorteilhaften Ausführungsform kann die Vorrichtung dazu konfiguriert sein, zumindest einen Teil des Flüssigkeitsüberschusses aus der Auffangvorrichtung dem Zerstäuber zum neuerlichen Zerstäuben zuzuführen. Durch eine derartige Rezirkulation kann ein größerer Anteil der im Behälter vorgelegten Flüssigkeit genützt und die maximal mögliche Anwendungsdauer mit einer Behälterfüllung erhöht werden.

Gemäß einer besonders vorteilhaften Weiterbildung kann der Behälter ein Druckbehälter sein, der ein mit dem komprimierten Gas befülltes Gaskompartment als Druckbeaufschlagungsmittel und ein mit der Flüssigkeit befülltes Flüssigkeitskompartment aufweist, wobei der Zerstäuber ein Ventil aufweist, so dass Flüssigkeit bei geöffnetem Ventil durch die Düse aus dem Flüssigkeitskompartment ausstoßbar ist, indem sich das Gaskompartment durch Expansion des Gases proportional zur Menge der ausgestoßenen Flüssigkeit vergrößert und das Flüssigkeitskompartment hierdurch um das Volumen der ausgestoßenen Flüssigkeit verkleinert, so dass eine maximal mögliche Volumenveränderung von Gaskompartment bzw. Flüssigkeitskompartment gegenüber einem bestimmten Anfangsfüllzustand von Gaskompartment bzw. Flüssigkeitskompartment für den bestimmten Anfangsfüllzustand eine maximale Ausstoßmenge an Flüssigkeit definiert.

Gemäß einer vorteilhaften Ausführungsform kann der Druck des komprimierten Gases im Anfangsfüllzustand so hoch sein, dass der Druck im Flüssigkeitskompartment vor Ausstoß der maximalen Ausstoßmenge an Inhalationsflüssigkeit 13 bar (1,3 MPa) nicht unterschreitet. Gemäß einer vorteilhaften Ausführungsform beträgt der Druck des komprimierten Gases im Anfangsfüllzustand mindestens 18 bar (1,8 MPa), vorzugsweise mindestens 20 bar (2 MPa), besonders bevorzugt mindestens 25 bar (2,5 MPa).

Auch bei Ausführungen in welchen Drücke im Flüssigkeitskompartment im Laufe des Ausstoßvorgangs 13 bar (1,3 MPa) unterschreiten oder auch während des gesamten Ausstoßvorgangs unter 13 bar (1,3 Mpa) ist es durch den freien Tropfenzerfall in Verbindung mit dem Prallelement auf für den Fachmann überraschende Weise möglich, über die gesamte Anwendungsdauer für die Erzeugung lungengängiger Tröpfchen geeignete Zerstäubungsparameter aufrechtzuerhalten. Derartige Ausführungsformen stellen aufgrund der niedrigeren Drücke geringere Anforderungen an die Fertigung von Druckbehälter und Ventil und können daher besonders vorteilhaft sein. Eine Ausführungsform, bei welcher der Anfangsdruck der Flüssigkeit im Behälter bei 13 bar (1,3 MPa) oder darunter liegt, kann auch deshalb als besonders vorteilhaft angesehen werden, da ein entsprechend geringerer Austrittsdruck den freien Tropfenzerfall begünstigen kann.

Für die Auslegung kann der nach Ausstoß der maximalen Ausstoßmenge an Flüssigkeit verbleibende Restdruck durch Auslegung mittels des allgemeinen Gasgesetzes erfolgen. Dabei sind erfindungsgemäß die Volumina des Gaskompartments und des Flüssigkeitskompartments so miteinander gekoppelt (beispielsweise über einen beide Kompartments voneinander trennenenden beweglichen Kolben), dass durch die maximale Füllmenge des Flüssigkeitskompartments mit physiologisch wirksamer Flüssigkeit ein maximales (Anfangs-)Volumen des Flüssigkeitskompartments und hierdurch ein minimales (Anfangs-)Volumen $V_1$ des Gaskompartments vorgegeben sind. Ebenfalls vorgegeben ist (beispielsweise mittels eines Anschlags) das minimale (End-) Volumen des Flüssigkeitskompartments nach Ausstoß der entsprechend maximalen Ausstoßmenge an Inhalationsflüssigkeit und durch die Kopplung das maximale (End-) Volumen $V_2$ des Gaskompartments. Der Enddruck $p_2$ im Gaskompartment entspricht dem minimalen Druck im Flüssigkeitskompartment bis zum Ausstoß der maximalen Ausstoßmenge an Inhalationsflüssigkeit und die Differenz zwischen End- und Anfangsvolumen des Gaskompartments ohne Übersetzung der Differenz zwischen Anfangs- und Endvolumen des Flüssigkeitskompartments.

Nach dem Gesetz idealer Gase

$$p_1 \cdot V_1 = p_2 \cdot V_2$$

ist somit der Anfangsdruck des komprimierten Gases nach der Beziehung

$$p_1 = p_2 \cdot V_2 / (V_2 - V_{Fmax})$$

zu wählen, worin $V_{Fmax}$ das maximal ausstoßbare Volumen an Flüssigkeit ist.

Zur Auslegung wird für $p_2$ der geringstmögliche Druck gewählt, der für die vorgesehene Geometrie des Zerstäubers eine Tröpfchengrößenverteilung innerhalb der gewünschten Parameter (definiert z.B. über den Sauterdurchmesser $d_{32}$, d.h. das Sechsfache des Kehrwerts der spezifischen Oberfläche der Tröpfchen des Aerosols) liefert, was beispielsweise mittels einfacher Auslegungsversuche geschehen kann, und dann entsprechend der obigen Beziehung für die gewünschte, $V_{Fmax}$ entsprechende Anwendungsmenge an Inhalationsflüssigkeit ermittelt, wie hoch beim zur Verfügung stehenden Anfangsvolumen des Gaskompartments der Fülldruck zu sein hat.

Andersherum kann natürlich auch anhand des maximal verfügbaren Gasfülldrucks das erforderliche Endvolumen des Gaskompartments bestimmt werden, wenn ein bestimmter Restdruck eingehalten werden soll. Bei einer beispielsweise vorgegebenen Anwendungsmenge (= maximales Ausstoßvolumen) von 150 ml Flüssigkeit und 1,3 MPa minimalem Druck im Flüssigkeitskompartment (unmittelbar vor Ausstoß des letzten Tropfens) sowie einem beispielsweise maximal verfügbaren Befülldruck von 3 MPa für das Gaskompartment wäre nach Umformung der obigen Beziehung das Endvolumen des Gaskompartments mit

$$V_2 = 150 \text{ ml} / (1 - 1{,}3 \text{ MPa} / 3 \text{ Mpa}) = 264{,}7 \text{ ml}$$

zu bemessen.

Gemäß einer vorteilhaften Weiterbildung kann das Ventil als Regulierventil ausgeführt sein, mittels welchem der Volumenstrom ausgestoßener Flüssigkeit regulierbar ist. Hierbei kann der Fachmann auf per se aus dem Stand der Technik bekannte Ventilkonstruktionen zurückgreifen.

Gemäß einer besonders vorteilhaften Weiterbildung kann die Vorrichtung eine Übersetzung aufweisen, welche den Druck im Flüssigkeitskompartment gegenüber dem Druck im Gaskompartment anhebt. Die oben erläuterte Auslegungsbeziehung ist dann entsprechend anzupassen, auch wenn Anfangs- und Endvolumina sowie Anfangs- und Enddrücke des Gaskompartments weiterhin auf Grundlage des allgemeinen Gasgesetzes ausgelegt werden können. Zu berücksichtigen ist dann jedoch ein Proportionalitätsfaktor zwischen der Volumenänderung im Gaskompartment und der Volumenänderung im Flüssigkeitskompartment sowie ein Übersetzungsverhältnis zwischen den Drücken im Gaskompartment und im Flüssigkeitskompartment.

Wird die Übersetzung vorteilhaft mit Hilfe eines das Gaskompartment auf einer Seite begrenzenden Gaskolbens mit einer gaskompartmentseitigen Gaskompartmentkolbenfläche und eines das Flüssigkeitskompartment auf einer Seite begrenzenden Flüssigkeitskolbens mit einer flüssigkeitskompartmentseitigen Flüssigkeitskompartmentkolbenfläche umgesetzt, wobei der Flächeninhalt $A_F$ der Flüssigkeitskompartmentkolbenfläche kleiner ist, als der Flächeninhalt $A_G$ der Gaskompartmentkolbenfläche, so ergibt sich ein Übersetzungsverhältnis k zwischen dem Druck im Flüssigkeitskompartment und dem Druck im Gaskompartment von

$$k = A_G : A_F$$

und als Volumenänderung im Gaskompartment das k-fache der jeweils ausgestoßenen Inhalationsflüssigkeit.

Für die Auslegung des Gaskompartments gilt dann $p_2 \geqq 1{,}3 \text{ Mpa} / k$ und

$$p_1 = p_2 \cdot V_2 / (V_2 - k \cdot V_{Fmax})$$

mit (wie oben) $p_2$ := Enddruck im Gaskompartment; $p_1$ := anfänglicher Fülldruck im Gaskompartment; $V_{Fmax}$ := maximal ausstoßbares Volumen an Inhalationsflüssigkeit; $V_2$ := Endvolumen des Gaskompartments.

Obiges gilt, wenn die Wege von Gas und Flüssigkeitskolben so gekoppelt sind, dass eine bestimmte Verschiebung des einen eine gleichgroße Verschiebung des anderen Kolbens bewirkt. Über Hebelmechaniken, Getriebe oder dgl. können Kolben selbstverständlich auch anderweitig gekoppelt werden. Auch sind Übersetzungsmechaniken mit Spindeltrieben und ähnlichem umsetzbar.

Gemäß einer bevorzugten Ausführungsform ist der Flächeninhalt der Flüssigkeitskompartmentkolbenfläche kleiner oder gleich der Hälfte des Flächeninhalts der Gaskompartmentkolbenfläche, so dass sich ein Übersetzungsverhältnis zwischen dem Druck im Flüssigkeitskompartment und dem Druck im Gaskompartment von $k \geqq 2$ ergibt, wenn die Wege von Gas und Flüssigkeitskolben so gekoppelt sind, dass eine bestimmte Verschiebung des einen eine gleichgroße Verschiebung des anderen Kolbens bewirkt.

Die Übersetzung zwischen Gaskompartment und Flüssigkeitskompartment bietet den Vorteil, dass höhere Flüssigkeitsdrücke mit geringeren Gasdrücken erzeugt werden können, so dass der Druckbehälter nur für geringere Gasdrücke ausgelegt werden muss, und die Sicherheit erhöht werden kann. Denn hohe Flüssigkeitsdrücke bauen sich bereits bei minimaler Expansion (z.B. durch eine Undichtigkeit) ab, wohingegen hohe Gasdrücke ein Sicherheitsrisiko bis hin zur Explosionsgefahr darstellen können. Eine solche Übersetzung lässt sich vorteilhaft nicht nur für Vorrichtung zum Verabreichen physiologisch wirksamer Flüssigkeiten, insbesondere Inhalatoren, sondern auch für Sprühdosen aller Art umsetzen, wobei das Flüssigkeitskompartment auch durch ein Mehrstoffkompartment ersetzt werden kann. Entsprechend kann vorteilhaft eine Sprühdose, insbesondere auch eine Sprühdose mit einem Sprühkopf der aus DE 20 2017 002 851 U1, DE 20 2017 005 165 U1 oder EP 3351172 A1 bekannten Art, bereitgestellt werden, welche einen Druckbehälter zum Aufnehmen einer Sprühflüssigkeit oder eines zu sprühenden Mehrstoffgemischs und komprimierten Gases und einen Zerstäuber zum Zerstäuben der Sprühflüssigkeit oder des zu sprühenden Mehrstoffgemischs aufweist, wobei der Druckbehälter ein mit dem komprimierten Gas befülltes Gaskompartment und ein mit der Sprühflüssigkeit befülltes Flüssigkeitskompartment oder ein mit dem Mehrstoffgemisch befülltes Mehrstoffkompartment aufweist, wobei der Zerstäuber ein Ventil und mindestens eine Düse aufweist, durch welche Sprühflüssigkeit oder Mehrstoffgemisch bei geöffnetem Ventil aus dem Flüssigkeitskompartment ausstoßbar ist, indem sich das Gaskompartment durch Expansion des Gases vergrößert und das Flüssigkeitskompartment oder Mehrstoffkompartment hierdurch um das Volumen der ausgestoßenen Sprühflüssigkeit oder des ausgestoßenen Mehrstoffgemischs verkleinert, wobei eine Übersetzung vorgesehen ist, welche den Druck im Flüssigkeitskompartment bzw. Mehrstoffkompartment gegenüber dem Druck im Gaskompartment anhebt.

Ein Zerstäuber mit mindestens einer Düse, durch welche Flüssigkeit aus dem Behälter ausstoßbar ist, und einem funktional mit der Düse kombinierten düsenaustrittsseitigen Prallelement, der so ausgelegt ist, dass in einem mit dem Druckbeaufschlagungsmittel erzeugbaren Druckbereich aus der Düse austretende Flüssigkeit vor dem Auftreffen auf das Prallelement in Tröpfchen zerfällt, zeichnet sich durch besonders gute Zerstäubung auch in niedrigeren Druckbereichen aus. Bei Anwendung des obigen Auslegungskriteriums, wonach der Druck des komprimierten Gases im Anfangsfullzustand so hoch ist, dass der Druck im Flüssigkeitskompartment vor Ausstoß der maximalen Ausstoßmenge an Inhalationsflüssigkeit 13 bar (1,3 MPa) nicht unterschreitet, können aber auch alternative Zerstäuber besonders vorteilhaft sein. Entsprechend kann vorteilhaft ganz allgemein ein Inhalator bereitgestellt werden, welcher einen Druckbehälter zum Aufnehmen von Inhalationsflüssigkeit und komprimiertem Gas, einen Zerstäuber zum Zerstäuben der Inhalationsflüssigkeit und einen Applikator zum Verabreichen zerstäubter Inhalationsflüssigkeit aufweist, wobei der Druckbehälter ein mit dem komprimierten Gas befülltes Gaskompartment und ein mit der Inhalationsflüssigkeit befülltes Flüssigkeitskompartment aufweist, der Zerstäuber ein Ventil und mindestens eine Düse aufweist, durch welche Inhalationsflüssigkeit bei geöffnetem Ventil aus dem Flüssigkeitskompartment ausstoßbar ist, indem sich das Gaskompartment durch Expansion des Gases proportional zur Menge ausgestoßener Inhalationsflüssigkeit vergrößert und das Flüssigkeitskompartment hierdurch um das Volumen der ausgestoßenen Inhalationsflüssigkeit verkleinert, so dass eine maximal mögliche Volumenveränderung von Gaskompartment bzw. Flüssigkeitskompartment gegenüber einem bestimmten Anfangsfüllzustand von Gaskompartment bzw. Flüssigkeitskompartment für den bestimmten Anfangsfüllzustand eine maximale Ausstoßmenge an Inhalationsflüssigkeit definiert, wobei der Druck des komprimierten Gases im Anfangsfüllzustand so hoch ist, dass der Druck im Flüssigkeitskompartment vor Ausstoß der maximalen Ausstoßmenge an Inhalationsflüssigkeit 13 bar (1,3 MPa) nicht unterschreitet. Auf für den Fachmann überraschende Weise ist es so möglich, durch geeignetes Abstimmen der Füllvolumina des Gaskompartments und des Flüssigkeitskompartments sowie des Anfangsfülldrucks des Gaskompartments über die gesamte Anwendungsdauer für die Erzeugung lungengängiger Tröpfchen geeignete Zerstäubungsparameter aufrechtzuerhalten.

Gemäß einer bevorzugten Ausführungsform des Inhalators mit alternativem Zerstäuber ist die mindestens eine Düse ausgeführt als mehrere Rayleigh-Düsenlöcher, beispielsweise runde Löcher in einer Düsenplatte.

Es hat sich herausgestellt, dass sich mit gleichgroßen Düsenlöchern für Drücke über 1,3 MPa und (geringste) Lochdurchmesser im einstelligen Mikrometerbereich nahezu monodisperse Tröpfchen mit Durchmessern von im Mittel ca. dem 1,89-fachen des Lochdurchmessers erzeugen lassen.

Entsprechend kann der geringste Durchmesser jedes der Rayleigh-Düsenlöcher vorteilhafterweise 6 $\mu$m oder

weniger, vorzugsweise 3 $\mu$m oder weniger, besonders bevorzugt zwischen 1 $\mu$m und 3 $\mu$m betragen.

Unter einem geringsten Durchmesser einer Düse oder Düsenöffnung wird in der vorliegenden Anmeldung der geringste mögliche Abstand der beiden Schnittpunkte einer die Mittelachse des die Düsenöffnung bildenden durchströmbaren Kanals (Austrittskanals) orthogonal schneidenden Linie mit der Berandung des jeweiligen Austrittskanals verstanden. Ist beispielsweise ein Austrittskanal im wesentlichen zylindrisch jedoch nicht orthogonal zu einer ebenen Oberfläche gebohrt, welche die Berandung der Austrittsöffnung bildet, so ergibt sich eine elliptische Austrittsöffnung, deren kürzere Hauptachse dem geringsten Durchmesser des Austrittskanals entspricht.

Grundsätzlich bemisst sich die Zahl der Düsenlöcher vorteilhaft nach dem gewünschten Flüssigkeitsdurchsatz, wenn der Lochdurchmesser aufgrund der erwünschten Tröpfchengröße festliegt. Gemäß einer bevorzugten Ausführungsform des Inhalators mit alternativem Zerstäuber sind mindestens 20 Rayleigh-Düsenlöcher vorgesehen.

Gemäß einer weiteren vorteilhaften Weiterbildung des Inhalators mit alternativem Zerstäuber kann mehr als eine Düse vorgesehen sein, wobei die Düsen einzeln oder gruppenweise von der Zufuhr von Inhalationsflüssigkeit ausschließbar sind. So lässt sich der Volumenstrom an ausgestoßener Inhalationsflüssigkeit steuern, indem mehr oder weniger Düsen an der Zerstäubung beteiligt sind. Werden verschiedene Düsen, beispielsweise Düsen mit unterschiedlichen Austrittsdurchmessern verwendet, so lassen sich durch selektives Auswählen bestimmter Düsen oder Düsengruppen Zerstäubungsparameter wie Tröpfchengröße bzw. - größenverteilung einstellen. Unterschiedliche technische Umsetzungen sind hier vorteilhaft möglich, etwa gegeneinander verschiebbare Lochscheiben mit je nach Position unterschiedlicher Überdeckung der Löcher, separate Ventile für separate Düsen bzw. Düsengruppen etc. Auch kann eine Düsenplatte mit mehreren Düsenöffnungen von einer Verschlussplatte zu unterschiedlichen Anteilen überdeckt werden, so dass beispielsweise nur größere, nur kleinere oder größere und kleinere Düsenöffnungen anströmbar sind.

Gemäß einer weiteren vorteilhaften Ausführungsform des Inhalators mit alternativem Zerstäuber ist die Düse als mindestens eine Mehrstrahldüsenanordnung ausgeführt, insbesondere mit mindestens zwei Austrittskanälen, welche so angeordnet sind, dass aus jeweils mindestens zweien der Austrittskanäle austretende Sprühstrahle an einem von den Austrittsöffnungen beabstandeten jeweiligen Aufprallort zentral aufeinander treffen. Vorteilhafte Ausführungen für derartige Mehrstrahldüsen können im wesentlichen wie in EP 3351172 A1 offenbart oder ähnlich ausgeführt werden.

Aufgrund der wie oben definierten Druckverhältnisse (hoher Restdruck) lassen sich Ausführungsformen des Inhalators mit alternativem Zerstäuber, bei welchen der geringste Durchmesser (Durchmesser des Austrittskanals gemäß obiger Erläuterung) der mindestens einen Düse 50 $\mu$m oder weniger, vorzugsweise 20 $\mu$m oder weniger, besonders bevorzugt 10 $\mu$m oder weniger beträgt, besonders vorteilhaft umsetzen. Bei Mehrstrahldüsen der genannten Art können Tröpfchengrößen durch Erhöhung des Drucks und Verminderung der Durchmesser der Austrittskanäle verringert werden.

Gemäß einer weiteren vorteilhaften Ausführungsform des Inhalators mit alternativem Zerstäuber ist die Düse als mindestens eine Hohlkegeldüse ausgeführt. Vorteilhafte Ausführungen für derartige Mehrstrahldüsen können im wesentlichen wie in DE 20 2017 005 165 U1 offenbart oder ähnlich (d.h. auch mit Abweichungen wie insbesondere einem im wesentlichem axialem Flüssigkeitseintritt) ausgeführt werden.

Aufgrund der wie oben definierten Druckverhältnisse (hoher Restdruck) lassen sich Ausführungsformen des Inhalators mit alternativem Zerstäuber, bei welchen der geringste Durchmesser des Austritts der Hohlkegeldüse (gemäß obiger Erläuterung) 100 $\mu$m oder weniger, vorzugsweise 50 $\mu$m oder weniger, besonders bevorzugt 20 $\mu$m oder weniger beträgt, besonders vorteilhaft umsetzen. Bei Hohlkegeldüsen mit tangentialem Flüssigkeitseintritt sorgt das erfindungsgemäße Druckkriterium dafür, dass unerwünschte sogenannte Tulpenbildung im Düsenkörper vermieden wird.

Alternativ zu einem mit komprimiertem Gas befüllten Gaskompartment können bei der erfindungsgemäßen tragbaren Vorrichtung vorteilhafterweise eine Handpumpe, eine insbesondere batteriebetriebene elektrische Pumpe oder ein Federsystem, wie für sich betrachtet aus dem Stand der Technik bekannt, als Druckbeaufschlagungsmittel dienen. Mit derartigen alternativen Druckbeaufschlagungsmitteln lässt sich die Vorrichtung besonders vorteilhaft derart konfigurieren, dass zumindest ein Teil des Flüssigkeitsüberschusses aus der Auffangvorrichtung dem Zerstäuber zum neuerlichen Zerstäuben zugeführt werden kann. Es braucht hierfür lediglich ein Rückströmungskanal, vorzugsweise mit einer Sieb- oder Filtereinrichtung, vorgesehen zu werden, über welchen abgetropfter Flüssigkeitsüberschuss beispielsweise der Pumpe zugeführt wird.

Die Herstellung der Düse kann bei der vorliegenden Erfindung wie auch bei dem Inhalator mit alternativem Zerstäuber vorteilhafterweise, wie per se aus dem Stand der Technik bekannt, lithographisch, durch Elektroerodieren oder mittels Sintern in Keramik oder Kunststoff hergestellt sein. Beim lithographischen Verfahren werden aus einem Siliziumdüsenkörper feine Kanäle herausgeätzt. Derartige Herstellungsverfahren sind im Zusammenhang mit Zerstäuberpumpen bzw. zugehörigen Sprühköpfe beispielsweise aus EP 1493492 A1 und EP 1386670 A2 bekannt.

Gemäß einer besonders vorteilhaften Weiterbildung der Erfindung wie auch des Inhalators mit alternativem Zerstäuber kann die mindestens eine Düse mittels Lasereinwirkung hergestellt sein, wobei vorzugsweise zum Bilden der Düse ein Düsenmaterial, vorzugsweise Quarzglas oder ein anderes geeignetes Glasmaterial, lokal einer Lasereinwirkung ausgesetzt wird und anschließend das der Lasereinwirkung ausgesetzte Düsenmateriel weggeätzt wird. Das Wegätzen von der Lasereinwirkung ausgesetztem Quarzglas kann beispielsweise unter Verwendung von Flusssäure (HF) oder vorzugsweise Kalilauge (KOH) erfolgen. Das Quarzglas kann auch geeignet dotiert sein, um die Laserbearbeitbarkeit zu verbessern. Ein solches Fertigungsverfahren ist per se unter anderem unter der Bezeichnung SLE *(Selective, Laser-Induced Etching,* selektives laserinduziertes Ätzen) bekannt und in Hermans, M. et al.: Selective, Laser-Induced Etching of Fused Silica at High Scan-Speeds Using KOH, JLMN-Journal of Laser Micro/Nanoengineering Vol. 9, No. 2, 2014 veröffentlicht. Fertigungsmaschinen, mit denen nach dem SLE-Verfahren gefertigt werden kann, sind unter der Bezeichnung LightFab Microscanner kommerziell erhältlich.

Gemäß einer vorteilhaften Ausführung der Erfindung ist die Vorrichtung eine Inhalationsvorrichtung, die Flüssigkeit eine Inhalationsflüssigkeit, und der Applikator weist ein Mundstück oder eine Mund- und/oder Nasen-Maske auf.

Für die Raucherentwöhnung oder als Rauchwarenersatz kann der Applikator ein Mundstück aufweisen und die Inhalationsflüssigkeit nikotinhaltig sein. Anders als bei bekannten E-Zigaretten erfolgt die Zerstäubung so vorzugsweise rein mechanisch ohne Erhitzen, so dass unerwünschte chemische Prozesse vermieden werden können.

Gemäß einer weiteren vorteilhaften Ausführungsform der Erfindung kann die Vorrichtung als eine ophthalmologische Vernebelungsvorrichtung ausgeführt sein, und der Applikator eine Augenmaske aufweisen. So können Wirkstoffe der Augenheilkunde vorteilhaft als Aerosol appliziert werden, oder es kann das Problem trockener Augen durch Flüssigkeitszugabe als Aerosol gemildert werden. Grundsätzlich ist die Applikation eines Aerosols für den Nutzer wesentlich angenehmer im Vergleich zur Gabe herkömmlicher Augentropfen, da der Kopf nicht in den Nacken gelegt werden muss, und die Dosierung sich wesentlich vereinfacht.

Gemäß einer weiteren vorteilhaften Ausführungsform der Erfindung wie auch des Inhalators mit alternativem Zerstäuber ist der mindestens einen Düse düseneintrittseitig eine Siebanordnung vorgeschaltet. Gerade bei kleinen Austrittskanälen (Düsenöffnungen) kann ansonsten die Gefahr bestehen, dass diese ganz oder teilweise verstopfen. Dabei kann die Siebanordnung vorteilhaft einstückig mit einem Düsenkörper ausgebildet werden wie in DE 20 2017 002 851 U1 offenbart oder ähnlich, oder aber es kann ein separat montierter Siebkörper verwendet werden.

Gemäß einer weiteren vorteilhaften Weiterbildung kann bei der erfindungsgemäßen Vorrichtung mehr als eine Düse vorgesehen sein, wobei die Düsen einzeln oder gruppenweise von der Zufuhr von Inhalationsflüssigkeit ausschließbar sind. So lässt sich der Volumenstrom an ausgestoßener Inhalationsflüssigkeit steuern, indem mehr oder weniger Düsen an der Zerstäubung beteiligt sind.

Die Erfindung wird nachfolgend in beispielhafter Weise anhand der beigefügten schematischen Zeichnungen näher erläutert. Die Zeichnungen sind nicht maßstabsgetreu; insbesondere entsprechen Verhältnisse der einzelnen Abmessungen zueinander aus Gründen der Anschaulichkeit teilweise nicht den Abmessungsverhältnissen in tatsächlichen technischen Umsetzungen. Es werden mehrere bevorzugte Ausführungsbeispiele beschrieben, auf welche die Erfindung jedoch nicht beschränkt ist.

Grundsätzlich kann jede im Rahmen der vorliegenden Anmeldung beschriebene bzw. angedeutete Variante der Erfindung besonders vorteilhaft sein, je nach wirtschaftlichen, technischen und ggf. medizinischen Bedingungen im Einzelfall. Soweit nichts gegenteiliges dargelegt ist, bzw. soweit grundsätzlich technisch realisierbar, sind einzelne Merkmale der beschriebenen Ausführungsformen austauschbar oder miteinander sowie mit per se aus dem Stand der Technik bekannten Merkmalen kombinierbar.

Es zeigt

Fig. 1a  die Querschnittsdarstellung einer erfindungsgemäßen, als Inhalator mit rohrförmigem Applikator ausgebildeten Vorrichtung mit einem beutelförmigen Flüssigkeitskompartment, wobei die Schnittebene der Fig. 1a in Fig. 1b als durchbrochene Linie B-B' angedeutet ist,

Fig. 1b  einen Ausschnitt einer zu Fig. 1a im Winkel von 90 Grad stehenden Querschnittsdarstellung des Inhalators aus Fig. 1a, wobei die Schnittebene der Fig. 1b in Fig. 1a als durchbrochene Linie A-A' angedeutet ist,

Fig. 2  den Ausschnitt einer Querschnittsdarstellung ähnlich Fig. 1b, wobei jedoch der Applikator des Inhalators maskenförmig ausgebildet ist,

Fig. 3a  den schematischen Aufbau einer erfindungsgemäßen Vorrichtung, bei welcher Prallelement und Düse einstückig in einem gemeinsame Bauelement ausgeführt sind, und Auffangreservoir sowie eine Inhalationsmaske jeweils abnehmbar sind,

Fig. 3b  einen schematischen Aufbau ähnlich Fig. 3a, wobei Prallelement und Auffangreservoir einstückig in einem gemeinsame Bauelement separat von der der Düse ausgeführt sind, und die Inhalationsmaske abnehmbar ist,

Fig. 4a  den schematischen Aufbau einer erfindungsgemäßen Vorrichtung ähnlich Fig. 3a, bei welcher Prallelement und Düse einstückig in einem gemeinsame Bauelement ausgeführt sind, und Auffangreservoir sowie eine Augenmaske ebenfalls einstückig in einem gemeinsamen Bauelement ausgeführt sind,

Fig. 4b     einen schematischen Aufbau ähnlich Fig. 4a, wobei Prallelement und Augenmaske separat von der Düse in einem gemeinsamen Bauelement zusammengefasst sind, und das Auffangreservoir ein Schwämmchen enthält,

Fig. 5     die Querschnittsdarstellung eines erfindungsgemäßen Inhalators ähnlich Fig. 1, wobei jedoch eine Übersetzung vorgesehen ist, die dem Flüssigkeitskompartment einen gegenüber dem Gaskompartment erhöhten Druck aufprägt,

Fig. 6     die Querschnittsdarstellung eines alternativen Inhalators mit einem beutelförmigen Flüssigkeitskompartment,

Fig. 7     den Ausschnitt einer Querschnittsdarstellung ähnlich Fig. 2, wobei jedoch ein alternativer Zerstäuber mit mehreren Düsenöffhungen vorgesehen ist,

Fig. 8     den Ausschnitt einer Querschnittsdarstellung ähnlich Fig. 1b, wobei jedoch ein alternativer Zerstäuber mit mehreren Düsenöffnungen und zusätzlich ein Schieber zum variablen Verdecken eines Teils der Düsenöffnungen vorgesehen ist, und

Fig. 9     die Querschnittsdarstellung eines alternativen Inhalators ähnlich Fig. 6, wobei jedoch das Flüssigkeitskompartment vom Gaskompartment mittels eines beweglichen Kolbens voneinander getrennt ist.

Einander entsprechende Elemente sind in den Zeichnungsfiguren jeweils mit den gleichen Bezugszeichen bezeichnet.

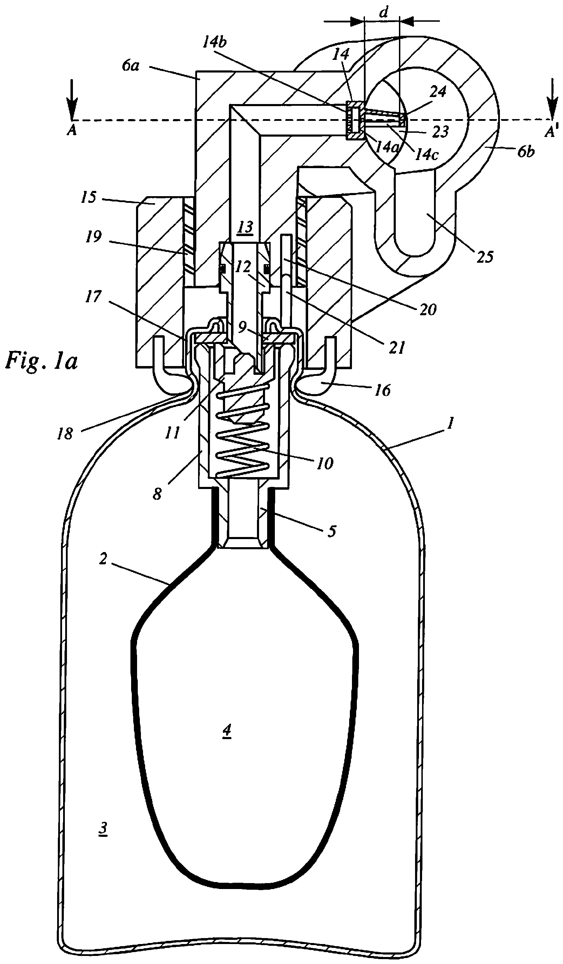

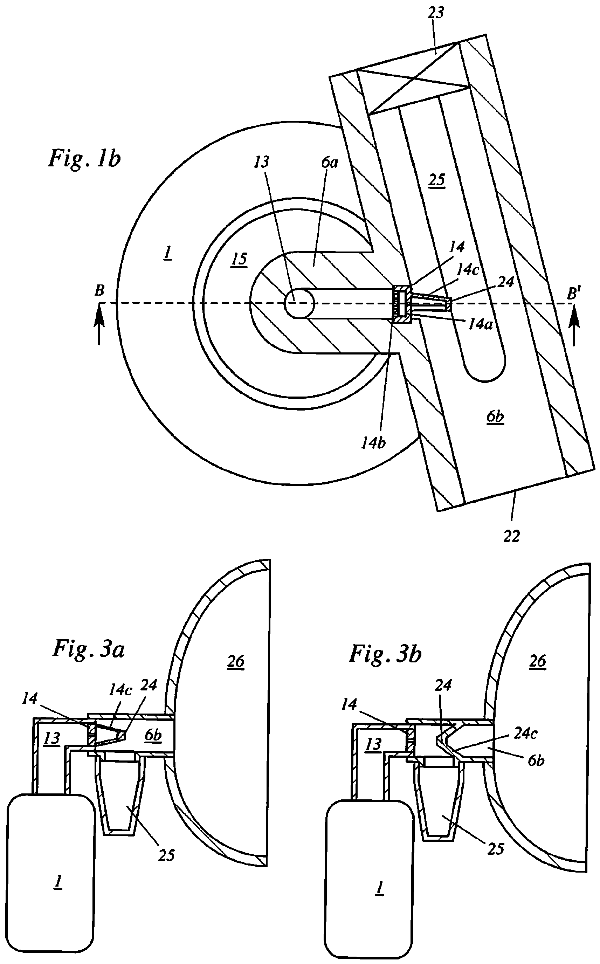

Der in Fig. 1a und 1b dargestellte Inhalator weist einen Druckbehälter 1 auf, welcher durch den flexiblen Beutel 2 in das mit komprimiertem Gas gefüllte Gaskompartment 3 und das Inhalationsflüssigkeit, beispielsweise Sole, aufweisende Flüssigkeitskompartment 4 unterteilt ist. Der Beutel 2 ist mit dem Eintrittsstutzen 5 der Zerstäuber-/Applikator-Einheit 6a, 6b verbunden, beispielsweise verschweißt oder verklebt. Anstelle eines Beutels kann auch beispielsweise ein beweiglicher Kolben das Gaskompartment 3 von dem Flüssigkeitskompartment 4 trennen.

Das Ventil des Zerstäubers 6a ähnelt üblichen Ventilen und weist ein Ventilgehäuse 8 auf, welches über den aus einem Elasten wie Kautschuk- oder Silikongummi bestehenden Dichtring 9 abgedichtet ist. Die ins Ventilgehäuse eingesetzte Feder 10 drückt die Verschlusskapsel 11 gegen den Dichtring 9. Durch Zusammendrücken von Hohlstößel 12 und Druckbehälter 1 relativ zu einander schiebt der unten angeschrägte Hohlstößel 12 die Verschlusskapsel 11 nach unten, so dass Inhalationsflüssigkeit durch Ventilgehäuse 8 und Hohlstößel 12 in den Zuführungskanal 13 der Düseneinheit 14 eintreten kann.

Am Druckbehälter 1 gehalten wird die Zerstäuber-/Applikator-Einheit 6a, 6b über einen Ring 15 mit um den Umfang des Rings gleichmäßig verteilten Rastnasen 16. Die Rastnasen 16 greifen in eine unterhalb des Kragens 17 umlaufende Einschnürung 18 des Druckbehälters 1 ein. Der Ring 15 ist über das Gewinde 19 drehbar mit der auf dem Hohlstößel 12 aufsitzenden Wandung des Zuführungskanals 13 verbunden. Aufgrund des in das Sackloch 20 der Wandung des Zuführungskanals 13 verschieblich eingreifenden, am Druckbehälter 1 festgeschweißten Stifts 21 ist die Wandung des Zuführungskanals 13 und somit der mit der Wandung fest verbundene Applikator 6b verdrehfest gegenüber dem Druckbehälter 1. Durch Drehen des Rings 15 lässt sich die Wandung des Zuführungskanals 13 zusammen mit dem Hohlstößel 12 kontrolliert auf und ab bewegen und so das Zusammendrücken von Hohlstößel 12 und Druckbehälter 1 relativ zueinander bewirken.

Durch die Düsenöffnungen des den Zuführungskanal 13 abschließenden Düsenkörpers 14 wird die Inhalationsflüssigkeit in den Innenraum des Applikators 6b hinein zerstäubt. Der Applikator 6b kann wie in Zusammenschau mit Fig. 1b erkennbar im wesentlichen röhrenförmig ausgeführt sein, so dass das offene Ende 22 der Röhre vom Mund des Anwenders zum Inhalieren umschlossen wird.

Mittels selektiver Laserbelichtung und anschließendem Ausätzen der belichteten Bereiche (selektives laserinduziertes Ätzen) ist in dem Düsenkörper 14 aus Quarzglas mit zylindrischer Grundform eine Düsenplatte 14a mit zentraler Austrittsöffnung sowie ein gegenüber der Austrittsöffnung angeordnetes Prallement 24 gebildet, ferner ein Siebkörper 14b mit einer Vielzahl von Sieböffnungen, deren Durchmesser höchstens dem Durchmesser der Austrittsöffnung entspricht, um Verstopfungen von letzterer fernzuhalten.

Das Prallelement 24 wird im vorliegenden Beispiel von drei in den Düsenkörper integrierten Streben 14c im Abstand d von der Austrittsöffnung gehalten (jeweils nur zwei der Streben 14c in Fig. 1a und Fig. 1b sichtbar), wobei in der Konstruktion selbstredend eine andere Zahl an Streben gewählt werden kann. Durch Auslegungsversuche sind der Behälterdruck (vorzugsweise 13 bar oder weniger im Flüssigkeitskompartment 4), der Durchmesser der Austrittsöffnung in der Düsenplatte 14a und der Abstand d zum Prallelement 24 so abgestimmt, dass der der Austrittsöffnung in der Düsenplatte 14a entweichende Flüssigkeitsstrahl vor dem Auftreffen auf das Prallelement 24 freiem Tropfenzerfall unterliegt.

Der Düsenkörper 14 kann eingegossen sein in ein einstückiges, die Wandung des Zuführungskanals 13 und den Applikator 6b umfassendes Kunststoff-Gussteil, er kann aber auch eingeklebt sein oder eingeklemmt zwischen zwei Bauteilen, wenn die Wandung des Zuführungskanals 13 und der Applikator 6b nicht gemeinsam einstückig ausgeführt werden.

Ist der Beutel 2 durch erfolgten Ausstoß der maximal ausstoßbaren Menge an Inhalationsflüssigkeit vollständig kollabiert, so hat das Gas im dann sein maximales Volumen aufweisenden Gaskompartment 3 einen gemäß gewünschter Auslegung gewählten Restdruck.

Figur 1b zeigt den Inhalator aus Fig. 1a in einer anderen, zu Fig. 1 a orthogonalen Schnittebene. Die Schnittebene der Fig. 1b ist in Fig. 1a als durchbrochene Linie A-A' angedeutet, die Blickrichtung des Betrachters durch Pfeile. Entsprechend ist die Schnittebene der Fig. 1a in Fig. 1b als durchbrochene Linie B-B' angedeutet, die Blickrichtung des Betrachters wiederum durch Pfeile.

Gegenüber dem offenen Ende 22 des Applikators 6b, das vom Mund des Anwenders zum Inhalieren umschlossen wird., schließt ein Einwegventil oder gasdurchlässiger Stopfen 23 das andere Ende des Applikators 6b ab.

Wie in der Zusammenschau von Fig. 1a und Fig. 1b erkennbar, weist der Applikator an seiner Unterseite eine Nut 25 auf, die sich in Richtung Stopfen 23 vertieft und Richtung offenes Ende 22 verflacht. Die Nut 25 dient als Auffangreservoir von Flüssigkeit, die vom Prallelement 24 abtropft, und ist auf der Seite des Stopfens 23 verschlossen.

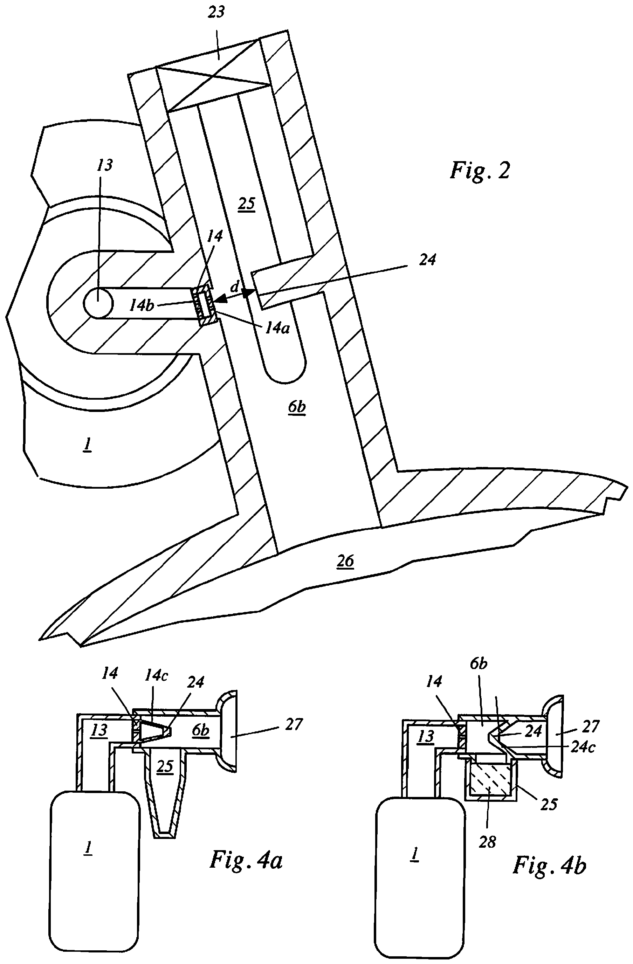

Fig. 2 zeigt, in ausgebrochener Darstellung, einen Inhalator ähnlich Fig. 1b, jedoch mit einem an seinem freien Ende als Maske 26 ausgebildeten Applikator 6b. Die (ausgebrochen dargestellt) Maske 26 kann zum Inhalieren über Mund und Nase des Anwenders gelegt werden und ist vorzugsweise ganz oder teilweise aus einem flexiblen Kunststoff oder Silikonmaterial gefertigt. Wiederum ist eine in Richtung luftdurchlässigen Stopfen 23 vertiefte Nut 25 als Auffangreservoir von ungenutzter Inhalationsflüssigkeit vorgesehen.

Anders als in Figuren 1a, 1b ist das Prallelement 24 nicht einstückig mit dem Düsenkörper 14 ausgebildet sondern in die Wandung des Applikators 6b integriert. Ferner enthält der Düsenkörper 14 mehr als eine, z.B. wie dargestellt zwei Austrittsöffnungen. Die Zahl der Austrittsöffnungen ist ein zusätzlicher Parameter, über den Druckverlust und Flüssigkeitsdruchsatz angepasst werden können.

Verschiedene Anordnungsvarianten einer ähnlich Fig. 2 aufgebauten erfindungsgemäßen Vorrichtung sind in Figuren 3a, 3b, 4a und 4b dargestellt.

In Fig. 3a ist das Prallelement 24 wiederum in den Düsenkörper 14 integriert. Die Inhalationsmaske 26 (Mund-Nasen-Maske) ist auf die Applikatorröhre 6b aufgesteckt oder mit dieser verschraubt, verklebt oder verschweißt. Das unterhalb des Prallelements 24 angeordnete Auffangreservoir 25 ist lösbar (beispielsweise durch eine Steck-, Schraub- oder BajonettVerbindung) oder dauerhaft (beispielsweise durch Schweißen oder Kleben) mit der Applikatorröhre 6b verbunden. Vor allem, wenn der Applikator wiederverwendbar ausgeführt werden soll, kann eine lösbare Verbindung zwischen Applikatorröhre 6b und Auffangreservoir 25 vorteilhaft sein.

Die Ausführung in Fig. 3b entspricht der Ausführung aus Fig. 3a, allerdings ist das Prallelement 24 nicht in den Düsenkörper 14 integriert sondern mittels mehrerer Streben 24c in die Applikatorröhre 6b.

Bei der Vorrichtung in Fig. 4a sind eine Augenmaske 27 und das Auffangreservoir 25 einstückig mit der Applikatorröhre 6b in ein gemeinsames Bauteil integriert. Wie in Fig. 3a weist der Düsenkörper 14 mehrere Streben 14c auf, welche das Prallelement 24 halten.

Die Vorrichtung in Fig. 4b unterscheidet sich von der Vorrichtung aus Abbildung 4a dadurch, dass das Prallelement 24 nicht in den Düsenkörper 14 sondern mittels mehrerer Streben 24c in die Applikatorröhre 6b integriert ist. Das abnehmbare Auffangreservoir 25 enthält ein Schwämmchen 28. Das Schwämmchen 28 bietet den Vorteil, dass überschüssige Flüssigkeit sicher im Auffangreservoir verbleibt, auch wenn die Vorrichtung sehr schräg gehalten oder geschüttelt wird. Die Funktion des Schwämmchens kann auch ein anderes saugfähiges Material, beispielsweise ein Vlies, Webstoff, Wattebausch, Partikelkollektiv etc. übernehmen.

Die Verbindung zwischen der Wandung des des Zufuhrkanals 13 zur Düse 14 und der Applikatorröhre 6b kann in Figuren 3a-4b fest oder lösbar sein.

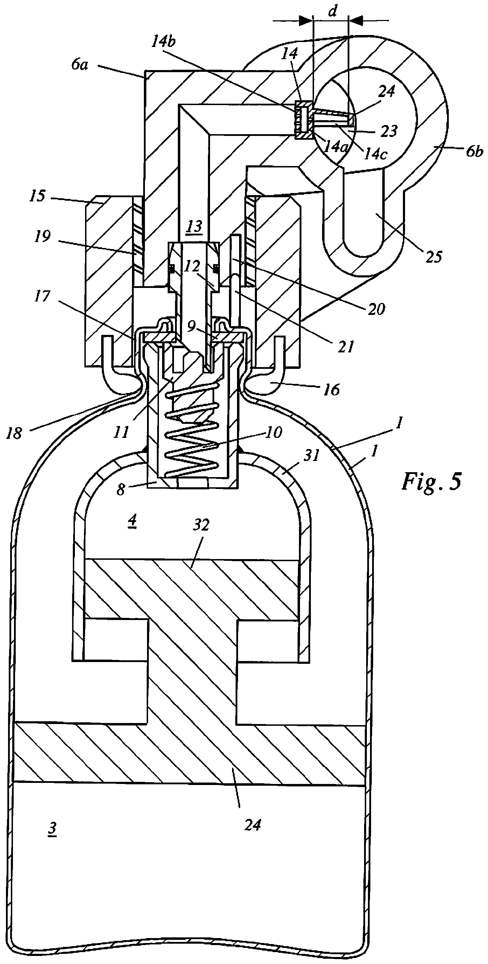

Bei dem in Fig. 5 dargestellten Inhalator ist die Zerstäuber-/Applikator-Einheit 6a, 6b aufgebaut wie in Fig. 1a. Hier ist das Flüssigkeitskompartment 4 durch ein inneres Gehäuse 31 und einen starr mit dem Gaskolben 24 verbundenen Flüssigkeitskolben 32 definiert. Die Einheit aus Gaskolben 24 und Flüssigkeitskolben 32 ist zum Ausstoß von Inhalationsflüssigkeit verschieblich.

Da die das Flüssigkeitskompartment 4 abschließende Fläche des Flüssigkeitskolbens 32 nur etwa den halben Flächeninhalt besitzt wie die das Gaskompartment 3 abschließende Fläche des Gaskolbens 24, ergibt sich ein Übersetzungsverhältnis von etwa 2, d.h. der Flüssigkeitsdruck im Flüssigkeitskompartment 4 ist etwa doppelt so hoch wie der gleichzeitige Gasdruck im Gaskompartment 3.

Unmittelbar bevor der Flüssigkeitskolben 32 durch erfolgten Ausstoß der maximal ausstoßbaren Menge an Inhalationsflüssigkeit seine Endposition erreicht, herrscht im Flüssigkeitskompartment 4 ein zuvor durch Auslegung definierter Restdruck, beispielsweise von 13 bar (1,3 MPa).

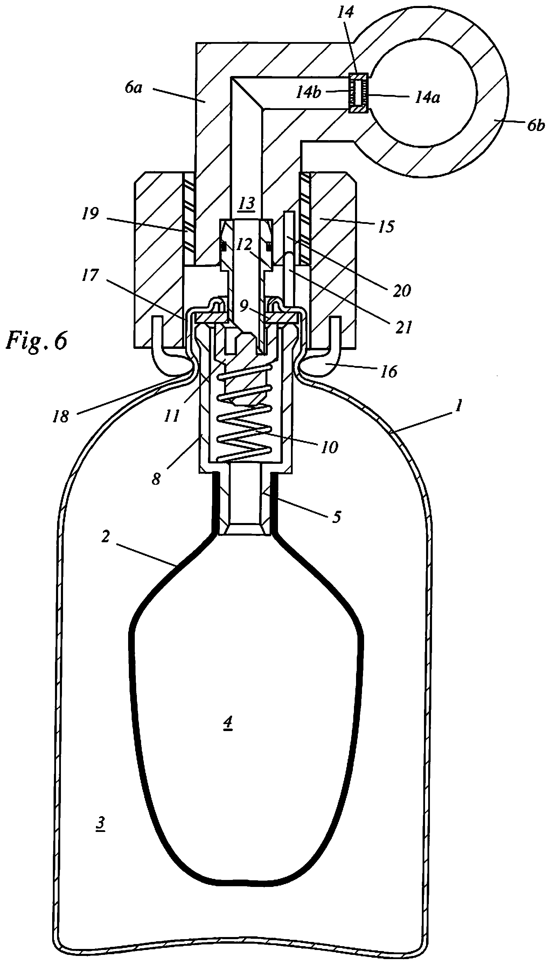

Der in Fig. 6 dargestellte Inhalator weist einen Druckbehälter 1 auf, welcher durch den flexiblen Beutel 2 in das mit komprimiertem Gas gefüllte Gaskompartment 3 und das Inhalationsflüssigkeit aufweisende Flüssigkeitskompartment 4 unterteilt ist. Der Beutel 2 ist mit dem Eintrittsstutzen 5 der (zum in Fig. 1 dargestellten Prinzip alternativen) Zerstäuber-/Applikator-Einheit 6a, 6b verbunden, beispielsweise verschweißt oder verklebt.

Das Ventil des Zerstäubers 6a ähnelt üblichen Ventilen und weist ein Ventilgehäuse 8 auf, welches über den aus einem Elasten wie Kautschuk- oder Silikongummi bestehenden Dichtring 9 abgedichtet ist. Die ins Ventilgehäuse eingesetzte Feder 10 drückt die Verschlusskapsel 11 gegen den Dichtring 9. Durch Zusammendrücken von Hohlstößel 12 und Druckbehälter 1 relativ zu einander schiebt der unten angeschrägte Hohlstößel 12 die Verschlusskapsel 11 nach unten, so dass Inhalationsflüssigkeit durch Ventilgehäuse 8 und Hohlstößel 12 in den Zuführungskanal 13 der Düseneinheit 14 eintreten kann.

Am Druckbehälter 1 gehalten wird die Zerstäuber-/Applikator-Einheit 6a, 6b über einen Ring 15 mit um den Umfang des Rings gleichmäßig verteilten Rastnasen 16. Die Rastnasen 16 greifen in eine unterhalb des Kragens 17 umlaufende Einschnürung 18 des Druckbehälters ein. Der Ring 15 ist über das Gewinde 19 drehbar mit der auf dem Hohlstößel 12 aufsitzenden Wandung des Zuführungskanals 13 verbunden. Aufgrund des in das Sackloch 20 der Wandung des Zuführungskanals 13 verschieblich eingreifenden, am Druckbehälter 1 festgeschweißten Stifts 21 ist die Wandung des Zuführungskanals 13 und somit der mit der Wandung fest verbundene Applikator 6b verdrehfest gegenüber dem Druckbehälter 1. Durch Drehen des Rings 15 lässt sich die Wandung des Zuführungskanals 13 zusammen mit dem Hohlstößel 12 kontrolliert auf und ab bewegen und so das Zusammendrücken von Hohlstößel 12 und Druckbehälter 1 relativ zu einander bewirken.

Durch die Düsenöffnungen des den Zuführungskanal 13 abschließenden Düsenkörpers 14 wird die Inhalationsflüssigkeit in den Innenraum des Applikators 6b hinein zerstäubt. Der Applikator 6b kann wie in Fig. 1b als Röhre ausgeführt sein, deren offenes Ende vom Mund des Anwenders zum Inhalieren umschlossen wird.

Mittels selektiver Laserbelichtung und anschließendem Ausätzen der belichteten Bereiche (selektives laserinduziertes Ätzen) ist in dem Düsenkörper 14 aus Quarzglas mit zylindrischer Grundform eine Düsenplatte 14a mit mehreren Austrittsöffnungen gebildet, ferner ein Siebkörper 14b mit einer Vielzahl von Sieböffnungen, deren Durchmesser in etwa dem Durchmesser der Austrittsöffnungen entspricht, um Verstopfungen von letzteren fernzuhalten.

Der Düsenkörper 14 kann eingegossen sein in ein einstückiges, die Wandung des Zuführungskanals 13 und den Applikator 6b umfassendes Kunststoff-Gussteil, er kann aber auch eingeklebt sein oder eingeklemmt zwischen zwei Bauteilen, wenn die Wandung des Zuführungskanals 13 und der Applikator 6b nicht gemeinsam einstückig ausgeführt werden.

Ist der Beutel 2 durch erfolgten Ausstoß der maximal ausstoßbaren Menge an Inhalationsflüssigkeit vollständig kollabiert, so hat das Gas im dann sein maximales Volumen aufweisenden Gaskompartment 3 einen zuvor durch Auslegung definierten Restdruck, beispielsweise von 13 bar (1,3 MPa).

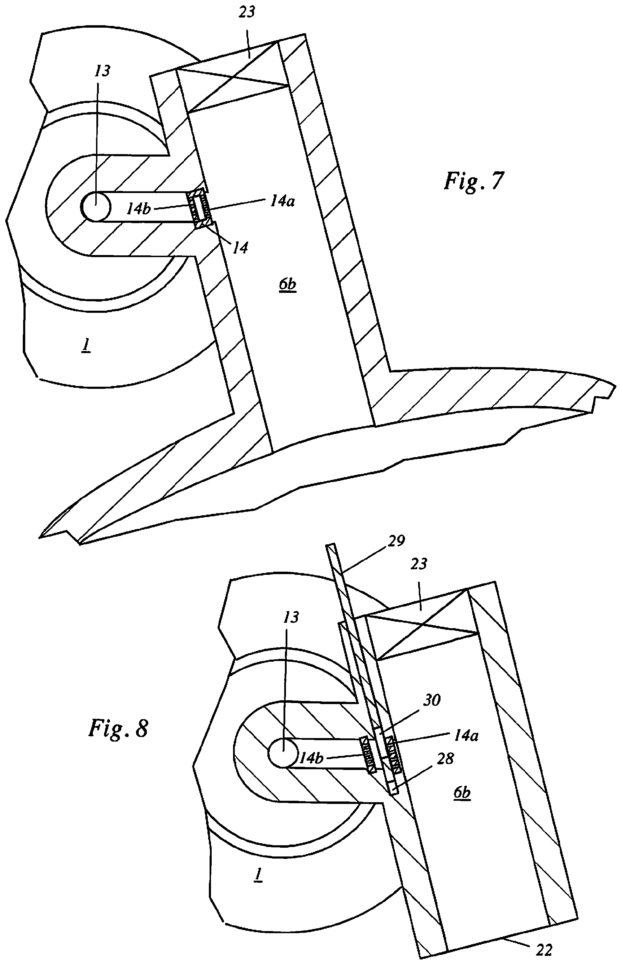

Fig. 7 zeigt, in ausgebrochener Darstellung ähnlich Fig. 2, einen Inhalator ähnlich Fig. 6, jedoch mit einem als Maske ausgebildeten Applikator 6b. Die Maske kann zum Inhalieren über Mund und Nase des Anwenders gelegt werden und ist vorzugsweise ganz oder teilweise aus einem flexiblen Kunststoff oder Silikonmaterial gefertigt.

Fig. 8 zeigt, in ausgebrochener Darstellung, einen Inhalator ähnlich Fig. 6. Allerdings sind hier die Düsenplatte 14a und der Siebkörper 14b als separate Bauteile ausgeführt, die separat in die Einheit aus Wandung des Zuführungskanals 13 und Applikator 6b eingegossen, eingeklebt oder anderweitig eingesetzt sind. Auch hier können Düsenplatte 14a und der Siebkörper 14b mittels selektiven laserinduzierten Ätzens aus Quarzglas hergestellt werden, aufgrund der jeweils einfacheren Gestaltung sind jedoch auch andere Herstellungsverfahren möglich, wie z.B. Bohren mittels (insb. Femtosekunden-)Lasers, lithographische Herstellung usw.

Ferner ist hier ein in einem gedichteten Spalt 28 verschieblicher Schieber 29 vorgesehen. Je nach Positionierung der Schieber-Apertur 30 ist ein Teil der Düsenöffnungen in der Düsenplatte 14a verdeckt. Zum einen kann so der Volumenstrom an austretender Inhalationsflüssigkeit kontrolliert werden. Zum anderen kann die Tröpfchengrößenverteilung verändert werden, wenn die Düsenöffnungen unterschiedlich groß sind und derart angeordnet, dass je nach Stellung des Schiebers 29 bzw. Position der Schieber-Apertur 30 unterschiedlich große Düsenöffnungen verdeckt sind bzw. freiliegen.

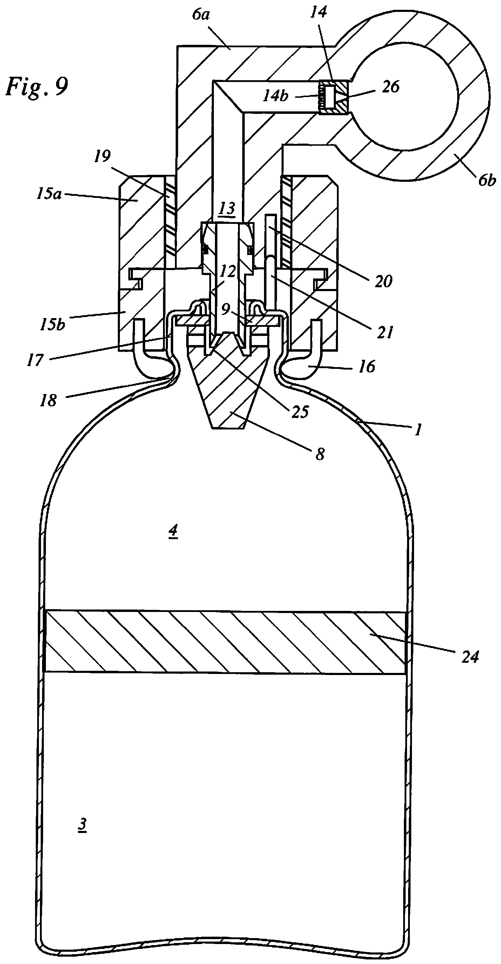

Fig. 9 zeigt wiederum einen alternativen Inhalator in Querschnittdarstellung ähnlich Fig. 6. Der dargestellte Inhalator weist wiederum einen Druckbehälter 1 auf, welcher durch den beweglichen Kolben 24 in das mit komprimiertem Gas gefüllte Gaskompartment 3 und das Inhalationsflüssigkeit aufweisende Flüssigkeitskompartment 4 unterteilt ist. Das Flüssigkeitskompartment 4 ist mit dem Ventilgehäuse 5 der Zerstäuber-/Applikator-Einheit 6a, 6b verbunden, welches einen kegeligen Ventilsitz 25 aufweist.

Durch Anheben des Hohlstößels 12 relativ zum Druckbehälter 1 entsteht ein Spalt zwischen dem konischen Ende des Hohlstößels 12 und dem Ventilsitz 25, so dass Inhalationsflüssigkeit durch Ventilgehäuse 8 und Hohlstößel 12 in den Zuführungskanal 13 der Düseneinheit 14 eintreten kann.

Am Druckbehälter 1 gehalten wird die Zerstäuber-/Applikator-Einheit 6a, 6b über einen zweiteiligen Ring 15a, 15b mit um den Umfang des unteren Teils 15b des Rings 15a, 15b gleichmäßig verteilten Rastnasen 16. Die Rastnasen 16 greifen in eine unterhalb des Kragens 17 umlaufende Einschnürung 18 des Druckbehälters 1 ein. Der obere Teil 15a des Rings 15a, 15b ist über das Gewinde 19 drehbar mit der auf dem Hohlstößel 12 aufsitzenden Wandung des Zuführungskanals 13 verbunden. Aufgrund des in das Sackloch 20 der Wandung des Zuführungskanals 13 verschieblich eingreifenden, am Druckbehälter 1 festgeschweißten Stifts 21 ist die Wandung des Zuführungskanals 13 und somit der mit der Wandung fest verbundene Applikator 6b verdrehfest gegenüber dem Druckbehälter 1. Durch Drehen des oberen Teils 15a des Rings 15a, 15b lässt sich die Wandung des Zuführungskanals 13 zusammen mit dem Hohlstößel 12 kontrolliert auf und ab bewegen und so das Ventil am Ventilsitz 25 kontrolliert öffnen und schließen.

Durch Einstellen des Ventilspalts zwischen dem Ventilsitz 25 und der passenden konischen Gegenfläche am unteren Ende des Hohlstößels 12 mittels Drehen am oberen Teil 15a des Rings 15a, 15b kann der Volumenstrom an austretender Inhalationsflüssigkeit reguliert werden.

Durch die Düsenöffnung 26 des den Zuführungskanal 13 abschließenden Düsenkörpers 14 wird die Inhalationsflüssigkeit in den Innenraum des Applikators 6b hinein zerstäubt. Der Applikator 6b kann wiederum wie in Fig. 8 als Röhre ausgeführt sein, deren offenes Ende vom Mund des Anwenders zum Inhalieren umschlossen wird.

Mittels selektiver Laserbelichtung und anschließendem Ausätzen der belichteten Bereiche (selektives laserinduziertes Ätzen) ist in dem Düsenkörper 14 aus Quarzglas mit zylindrischer Grundform ein zylindrisch-konischer Hohlraum 27 gebildet. Eintrittsseitig weist der Düsenkörper einen Siebkörper 14b mit einer Vielzahl von Sieböffnungen auf, deren Durchmesser in etwa dem Durchmesser der Austrittsöffnung 26 entspricht, um Verstopfungen von letzterer fernzuhalten. Der Düsenkörper 14 kann eingegossen sein in ein einstückiges, die Wandung des Zuführungskanals 13 und den Applikator 6b umfassendes KunststoffGussteil, er kann aber auch eingeklebt sein oder eingeklemmt zwischen zwei Bauteilen, wenn die Wandung des Zuführungskanals 13 und der Applikator 6b nicht gemeinsam einstückig ausgeführt werden.

Hat der Kolben 24 durch erfolgten Ausstoß der maximal ausstoßbaren Menge an Inhalationsflüssigkeit seine Endposition erreicht, so hat das Gas im dann sein maximales Volumen aufweisenden Gaskompartment 3 einen zuvor durch Auslegung definierten Restdruck, beispielsweise von 13 bar (1,3 MPa).

## Patentansprüche

1. Tragbare Vorrichtung zum Verabreichen einer physiologisch wirksamen Flüssigkeit, aufweisend einen Behälter zum Aufnehmen der Flüssigkeit, Druckbeaufschlagungsmittel zum Beaufschlagen der Flüssigkeit mit Druck, einen Zerstäuber (6a) zum Zerstäuben der Flüssigkeit und einen Applikator (6b) zum Verabreichen zerstäubter Flüssigkeit,

   wobei der Zerstäuber (6a) mindestens eine Düse (14), durch welche Flüssigkeit aus dem Behälter ausstoßbar ist, aufweist,
   **dadurch gekennzeichnet, dass**
   der Zerstäuber (6a) ein funktional mit der Düse (14) kombiniertes düsenaustrittsseitiges Prallelement (24) zur Prallzerstäubung aufweist, und
   wobei der Zerstäuber (6a) so ausgelegt ist, dass ein in einem mit dem Druckbeaufschlagungsmittel erzeugbaren Druckbereich aus der Düse (14) austretender Flüssigkeitsstrahl vor dem Auftreffen auf das Prallelement (24) in freiem Zerfall in Tröpchen zerfällt.

2. Tragbare Vorrichtung gemäß Anspruch 1, welche eine Auffangvorrichtung zum Auffangen eines von dem Prallelement (24) abtropfenden oder abfließenden Flüssigkeitsüberschusses aufweist.

3. Tragbare Vorrichtung gemäß Anspruch 2, wobei
   die Auffangvorrichtung und der Applikator (6b) und/oder das Prallelement (24) in ein gemeinsames Bauteil integriert sind.

4. Tragbare Vorrichtung gemäß einem der Ansprüche 2 oder 3, wobei die Auffangvorrichtung ein Auffangreservoir (25) aufweist und das Auffangreservoir (25) ein saugfähiges Material (28) aufweist.

5. Tragbare Vorrichtung gemäß einem der Ansprüche 2 oder 3, welche dazu konfiguriert ist, zumindest einen Teil des Flüssigkeitsüberschusses dem Zerstäuber (6a) zum neuerlichen Zerstäuben zuzuführen.

6. Tragbare Vorrichtung gemäß einem der vorangehenden Ansprüche, wobei der Behälter ein Druckbehälter (1) ist, der ein mit dem komprimierten Gas befülltes Gaskompartment (3) als Druckbeaufschlagungsmittel und ein mit der Flüssigkeit befülltes Flüssigkeitskompartment (4) aufweist,
   der Zerstäuber (6a) ein Ventil aufweist, so dass Flüssigkeit bei geöffnetem Ventil durch die Düse (14) aus dem Flüssigkeitskompartment (4) ausstoßbar ist, indem sich das Gaskompartment (3) durch Expansion des Gases proportional zur Menge der ausgestoßenen Flüssigkeit vergrößert und das Flüssigkeitskompartment (4) hierdurch um das Volumen der ausgestoßenen Flüssigkeit verkleinert, so dass eine maximal mögliche Volumenveränderung von Gaskompartment (3) bzw. Flüssigkeitskompartment (4) gegenüber einem bestimmten Anfangsfüllzustand von Gaskompartment (3) bzw. Flüssigkeitskompartment (4) für den bestimmten Anfangsfüllzustand eine maximale Ausstoßmenge an Flüssigkeit definiert.

7. Tragbare Vorrichtung gemäß Anspruch 6, wobei der Druck des komprimierten Gases im Anfangsfüllzustand so hoch ist, dass der Druck im Flüssigkeitskompartment (4) bis zum erfolgten Ausstoß der maximalen Ausstoßmenge an Inhalationsflüssigkeit 13 bar (1,3 MPa) nicht unterschreitet.

8. Tragbare Vorrichtung gemäß einem der Ansprüche 6 oder 7, wobei der Druck des komprimierten Gases im Anfangsfüllzustand mindestens 18 bar (1,8 MPa), vorzugsweise mindestens 20 bar (2 MPa), besonders bevorzugt mindestens 25 bar (2,5 MPa) beträgt.

9. Tragbare Vorrichtung gemäß einem der Ansprüche 6-8, aufweisend eine Übersetzung, welche den Druck im Flüssigkeitskompartment (4) gegenüber dem Druck im Gaskompartment (3) anhebt.

10. Tragbare Vorrichtung gemäß einem der Ansprüche 1-7, wobei das Druckbeaufschlagungsmittel eine Handpumpe oder elektrisch betriebene Pumpe oder ein Federsystem aufweist.

11. Tragbare Vorrichtung gemäß einem der Ansprüche 1-10, wobei die mindestens eine Düse (14) mittels Lasereinwirkung hergestellt ist.

12. Tragbare Vorrichtung gemäß Anspruch 11, wobei zum Bilden der Düse (14) ein Düsenmateriel lokal einer Lasereinwirkung ausgesetzt wird und anschließend das der Lasereinwirkung ausgesetzte Düsenmateriel weggeätzt wird.

13. Tragbare Vorrichtung gemäß einem der vorangehenden Ansprüche, wobei die Vorrichtung eine Inhalationsvorrichtung ist, die Flüssigkeit eine nikotinhaltige Inhalationsflüssigkeit ist, und der Applikator (6b) ein Mundstück aufweist.

14. Tragbare Vorrichtung gemäß einem der Ansprüche 1-12, wobei die Vorrichtung eine ophthalmologische Vernebelungsvorrichtung ist, und der Applikator eine Augenmaske (27) aufweist.

15. Tragbare Vorrichtung gemäß einem der vorangehenden Ansprüche, wobei der mindestens einen Düse (14a) düseneintrittseitig eine Siebanordnung (14b) vorgeschaltet ist.

## Claims

1. Portable device for administering a physiologically active liquid, comprising a container for holding the liquid, pressurizing means for applying pressure to the liquid, an atomizer (6a) for atomizing the liquid and an applicator (6b) for administering atomized liquid,

   wherein the atomizer (6a) comprises at least one nozzle (14) through which liquid can be ejected from the container,
   **characterized in that**
   the atomizer (6a) further comprises, on the nozzle outlet side, an impact element (24) for impact atomization, said impact element (24) being functionally combined with the nozzle (14), and
   wherein the atomizer (6a) is configured in such a way that, in a pressure range that can be generated with the pressurizing means, a liquid jet emerging from the nozzle (14) breaks up, under a free break-up regime, into droplets prior to striking the impact element (24).

2. Portable device according to claim 1, which has a collection device for collecting excess liquid dripping or running from the impact element (24).

3. Portable device according to claim 2, wherein the collection device and the applicator (6b) and/or the impact element (24) are integrated into a common component.

4. Portable device according to any of claims 2 or 3, wherein the collection device comprises a collection reservoir (25), and the collection reservoir (25) comprises an absorbent material (28).

5. Portable device according to any of claims 2 or 3, which is configured to supply at least part of the excess liquid to the atomizer (6a) for re-atomizing.

6. Portable device according to any of the preceding claims, wherein the container is a pressure tank (1) having a gas compartment (3) filled with compressed gas as a pressurizing means and a liquid compartment (4) filled with liquid, the atomizer (6a) has a valve such that liquid can be ejected from the liquid compartment (4) through the nozzle (14) when the valve is open, in that the gas compartment (3) increases proportionally to the quantity of liquid ejected by expansion of the gas and the liquid compartment (4) thereby decreases by the volume of the liquid ejected, such that a maximum possible change in volume of the gas compartment (3) or liquid compartment (4) compared to a specific initial filling state of the gas compartment (3) or liquid compartment (4), respectively, defines a maximum ejection quantity of liquid for the specific initial filling state.

7. Portable device according to claim 6, wherein the pressure of the compressed gas in the initial filling state is so high that the pressure in the liquid compartment (4) does not fall below 13 bar (1.3 MPa) until the maximum ejection quantity of inhalation liquid has been successfully ejected.

8. Portable device according to any of claims 6 or 7, wherein the pressure of the compressed gas in the initial filling state is at least 18 bar (1.8 MPa), preferably at least 20 bar (2 MPa), particularly preferably at least 25 bar (2.5 MPa).

9. Portable device according to any of claims 6-8, having a transmission which increases the pressure in the liquid compartment (4) compared to the pressure in the gas compartment (3).

10. Portable device according to any of claims 1-7, wherein the pressurizing means comprises a hand pump or an electrically operated pump or a spring system.

11. Portable device according to any of claims 1-10, wherein the at least one nozzle (14) is manufactured by means of laser action.

12. Portable device according to claim 11, wherein a nozzle material is locally exposed to laser action to form the nozzle (14), and the nozzle material exposed to the laser action is subsequently etched away.

13. Portable device according to any of the preceding claims, wherein the device is an inhalation device, the liquid is a nicotine containing inhalation liquid, and the applicator (6b) has a mouthpiece.

14. Portable device according to any of claims 1-12, wherein the device is an ophthalmic nebulizer and the applicator comprises an eye mask (27).

15. Portable device according to any of the preceding claims, wherein a screen arrangement (14b) is arranged upstream to the at least one nozzle (14a) on the nozzle inlet side.

**Revendications**

1. - Dispositif portable pour l'administration d'un liquide physiologiquement actif, présentant un conteneur pour contenir le liquide, un moyen de mise sous pression pour appliquer une pression au liquide, un atomiseur (6a) pour atomiser le liquide et un applicateur (6b) pour administrer le liquide atomisé,

   l'atomiseur (6a) présentant au moins une buse (14) à travers laquelle du liquide est apte à être éjecté du conteneur,
   **caractérisé par le fait que**
   l'atomiseur (6a) présente un élément d'impact (24) côté sortie de buse, fonctionnellement combiné avec la buse (14), pour la pulvérisation par impact, et
   l'atomiseur (6a) étant configuré de telle sorte qu'un jet de liquide sortant de la buse (14) dans une zone de pression apte à être générée par le moyen de mise sous pression se désintègre librement en gouttelettes avant de frapper l'élément d'impact (24).

**2.** - Dispositif portable selon la revendication 1, lequel présente un dispositif de collecte pour collecter un excédent de liquide s'égouttant ou s'écoulant de l'élément d'impact (24).

**3.** - Dispositif portable selon la revendication 2, dans lequel le dispositif de collecte et l'applicateur (6b) et/ou l'élément d'impact (24) sont intégrés dans un composant commun.

**4.** - Dispositif portable selon l'une des revendications 2 ou 3, dans lequel le dispositif de collecte présente un réservoir de collecte (25) et le réservoir de collecte (25) présente un matériau absorbant (28) .

**5.** - Dispositif portable selon l'une des revendications 2 ou 3, lequel est configuré pour amener au moins une partie de l'excédent de liquide à l'atomiseur (6a) pour la nouvelle atomisation.

**6.** - Dispositif portable selon l'une des revendications précédentes, dans lequel le conteneur est un conteneur sous pression (1) qui présente un compartiment de gaz (3) rempli du gaz comprimé comme moyen de mise sous pression et un compartiment de liquide (4) rempli du liquide,
l'atomiseur (6a) présentant une soupape, de telle sorte que du liquide est apte à être éjecté du compartiment de liquide (4) à travers la buse (14) lorsque la soupape est ouverte, en ce que le compartiment de gaz (3) s'agrandit par expansion du gaz proportionnellement à la quantité du liquide éjecté, et le compartiment de liquide (4) se réduit ainsi du volume du liquide éjecté, de telle sorte qu'un changement de volume maximum possible du compartiment de gaz (3) ou du compartiment de liquide (4) par rapport à un état de remplissage initial déterminé du compartiment de gaz (3) ou du compartiment de liquide (4) définit une quantité maximale d'éjection de liquide pour l'état de remplissage initial déterminé.

**7.** - Dispositif portable selon la revendication 6, dans lequel la pression du gaz comprimé dans l'état de remplissage initial est suffisamment élevée pour que la pression dans le compartiment de liquide (4) ne tombe pas au-dessous de 13 bars (1,3 MPa) jusqu'à ce que la quantité d'éjection maximale de liquide d'inhalation ait été éjectée avec succès.

**8.** - Dispositif portable selon l'une des revendications 6 ou 7, dans lequel la pression du gaz comprimé dans l'état de remplissage initial est d'au moins 18 bars (1,8 MPa), de préférence d'au moins 20 bars (2 MPa), de façon particulièrement préférée d'au moins 25 bars (2,5 MPa) .

**9.** - Dispositif portable selon l'une des revendications 6 à 8, présentant une transmission, laquelle augmente la pression dans le compartiment de liquide (4) par rapport à la pression dans le compartiment de gaz (3).

**10.** - Dispositif portable selon l'une des revendications 1 à 7, dans lequel le moyen de mise sous pression présente une pompe manuelle ou une pompe à commande électrique ou un système à ressort.

**11.** - Dispositif portable selon l'une des revendications 1 à 10, dans lequel ladite au moins une buse (14) est fabriquée par action laser.

**12.** - Dispositif portable selon la revendication 11, dans lequel un matériau de buse est localement exposé à une action laser pour former la buse (14), puis le matériau de buse exposé à l'action laser est gravé.

**13.** - Dispositif portable selon l'une des revendications précédentes, dans lequel le dispositif est un dispositif d'inhalation, le liquide est un liquide d'inhalation contenant de la nicotine et l'applicateur (6b) présente un embout buccal.

**14.** - Dispositif portable selon l'une quelconque des revendications 1 à 12, dans lequel le dispositif est un dispositif de nébulisation ophtalmologique et l'applicateur comprend un masque oculaire (27).

**15.** - Dispositif portable selon l'une quelconque des revendications précédentes, dans lequel un ensemble de tamisage (14b) est placé en amont de ladite au moins une buse (14a) du côté entrée de buse.

*Fig. 1a*

*Fig. 1b*

*Fig. 3a*

*Fig. 3b*

*Fig. 2*

*Fig. 4a*

*Fig. 4b*

*Fig. 5*

*Fig. 6*

Fig. 7

Fig. 8

*Fig. 9*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2016184761 A1
- DE 102014207657 B3
- US 4368850 A
- US 3838686 A
- DE 202017002851 U1
- DE 202017005165 U1
- EP 3351172 A1
- EP 1493492 A1
- EP 1386670 A2

**In der Beschreibung aufgeführte Nicht-Patentliteratur**

- **HERMANS, M. et al.** Selective, Laser-Induced Etching of Fused Silica at High Scan-Speeds Using KOH. *JLMN-Journal of Laser Micro/Nanoengineering,* 2014, vol. 9 (2